# EUROPEAN PATENT APPLICATION

(11) **EP 2 036 977 A1**
(43) Date of publication of application: **18.03.2009**
(21) Application number: 07788584.6
(22) Date of filing: 07.06.2007
(51) Int. Cl.: C12M 1/42, C12M 1/00, B01D 53/84

(54) **ENERGY PHOTOCONVERTER FOR OBTAINING BIOFUELS**

(30) Priority: 09.06.2006 ES 200601567
(71) Applicant: Stroïazzo-Mougin, Bernard A. J., 03560 El Campello (Alicante) (ES); Gomis Catala, Cristian, 03560 El Campello Alicante (ES)
(72) Inventor: Stroïazzo-Mougin, Bernard A. J., 03560 El Campello (Alicante) (ES); Gomis Catala, Cristian, 03560 El Campello Alicante (ES)
(74) Representative: Elzaburu Marquez, Alberto
(86) International application number: PCT/ES2007/000336
(87) International publication number: WO 2007/144440

(57) **Abstract**

The present invention relates to the design of energy photoconverters which act in a continuous and closed manner for producing biofuels and other products of interest by means of the mass culturing of phytoplankton.

## Description

### Technical Field of the Invention:

The present invention relates to the design of energy photoconverters acting in a continuous and closed manner for producing biofuels and other products of interest by means of the mass culturing of phytoplankton.

The invention is comprised within the technical field of the exploitation of renewable energy by means of the action of phytoplankton organisms that normally belong to the following taxonomic families: Chlorophyceae, Bacillariophyceae, Dinophyceae, Cryptophyceae, Chrysophyceae, Haptophyceae, Prasinophyceae, Raphidophyceae ... generally the taxonomic families comprising species of the chromophyte division, all of them characterized by being flagellated or nonflagellated single-celled organisms and with a strictly planktonic (holoplanktonic) life phase, or at least one of its phases being planktonic (meroplanktonic).

Particularly by means of the use of energy converters, products such as biofuels, byproducts such as naphthas, kerosene, thermal energy, electric energy, ... are obtained.

### State of the Art

Up until now, biofuels have been obtained from higher plant cultures, usually from the group of phanerogams or flowering plants (sunflowers, palm, European palm,..), and usually on the terrestrial surface (land plants).

The obligation for the economic zones to comply with the objectives imposed by the Kyoto protocol on the reduction of CO₂/SO₂ emissions and the emission of other gases causing the so-called greenhouse effect is forcing countries to search for alternative and renewable fuels to prevent possible penal taxes.

Although the production of solar and wind energy is increasing in some regions, these technologies are very expensive and are not viable in all climatic areas. In these conditions, biofuels have an important role as substitutes of fossil fuels, especially in transport and heating applications.

The production costs of biofuels from plants, such as palm and rapeseed oil have always been a reason for concern. Taking into account the low oil production indexes per hectare, enormous amounts of resources would be needed to reach commercial production. Land and water are two limited resources and it is preferable to use them to produce food products, which are furthermore more profitable for farmers. Intensive fertilization is furthermore a form of land and water pollution of the first order. Extensive single crop farming is also one of the main enemies of biodiversity.

Phytoplankton represents a viable solution to the previously discussed drawback given that about 50% of the dry mass of single-celled organisms is generally biofuel. In addition, the annual production per hectare of biofuel from phytoplankton is 40 times higher than with the second most cost-effective product, palm oil. A drawback is that the production of phytoplankton oil requires covering vast stretches of land with rather shallow water, as well as introducing large amounts of CO₂, an essential element for phytoplankton to produce oil. Natural production systems such as phytoplankton ponds, have a relatively low cost but the harvesting process is very laborious and, therefore expensive. On the other hand, phytoplankton culturing is carried out in open systems, making it vulnerable to contamination and to problems for cultures, which may lead to total production loss. In this same sense, an advantage of the photoconverter described in the present invention is that the system is kept closed and in conditions such that the culture is not contaminated by bacteria, fungi,... because in addition to being closed, the culture is enriched by means of nutrients incorporating fungicides and antibiotics.

Within the field of the design of photoconverters for producing biofuels through photosynthetic microorganisms, two types of photoconverters could be clearly differentiated: open photoconverters, in which a direct exchange of matter between the culture and the air surrounding it is allowed, and closed photoconverters, in which this exchange is eliminated by means of the placement of a transparent physical medium allowing the passage of electromagnetic radiation but not the exchange of matter. The open photoconverters have many problems derived from the little control of the culturing conditions and possible contaminations, so their application is limited due to these drawbacks. However, closed photoconverters efficiently reduce these problems by means of greater control of the culturing conditions and possible contaminations and can reach a production rate that is 400 times higher than the production rate of sunflower.

Until now no systems similar to the photoconverter object of the present invention have been described which incorporate the advantages of being a closed system with a large volume and large diameters, which works continuously, which allows obtaining large amounts of biofuels or byproducts such as naphthas, glycerin, silicon-derived compounds, such as ferrosilicates, which may further obtain thermal and electric energy that does not contaminate given that all the possible residues, such as carbon dioxide, are recirculated in the system to be used as a nutrient for the phytoplankton, or which recirculates the water used as part of the culture medium so it can be reused ... The present invention therefore describes a novel system (photoconverter) including all these features which allows enormous versatility and is very environmental friendly.

Patent application WO 03/094598 A1 entitled "Photobioreactor and process for biomass production and mitigation of pollutants in flue gases" describes a generic photobioreactor model mainly focused on decontaminating COx, SOx and NOx type gases. It is basically a system working in a discontinuous manner (distinguishing between day/night photoperiod) and is open, its liquid medium not being axenic. It does not control nitrogen and carbon dioxide concentrations for the purpose of increasing biofuel production. It is not designed to work with monospecific or monoclonal algae strains. Its design does not contemplate biofuel production as the main objective, rather it is focused on gas purification. On the other hand, as regards the photosynthetic organisms it refers to, it does not demand conditions disabling the system and it has no controlled recirculation because the transport is done by a turbulent flow of bubbles.

Compared to the present invention object of the patent, a completely novel system is set forth which is based, in contrast, on the following features:
- It is completely closed.
- It is completely axenic.
- It works continuously.
- It works with monospecific and monoclonal strains.
- It does not accept any photosynthetic organism, but rather it at least requires that they are not organisms forming biofouling on the inner surface of the photoconverter.
- It requires that the phytoplankton species do not form colonies.
- It requires that the phytoplankton species do not generate exo-mucilage.
- It requires that the cultured species contains at least 5% of fatty acids.
- It enhances the use of nonflagellated and floating phytoplankton species.
- It does not accept any type of liquids as culture medium, it focuses on freshwater, brackish water and sea water.
- Its main objective is to obtain metabolic synthesis compounds with energetic properties or with pre-energetic properties essentially aimed at obtaining biofuels.

### Description

The present invention relates to an energy photoconverter for obtaining biofuels and other, though not less important, byproducts. Said photoconverter uses a Tichelmann-type flow control system which allows providing equal pressure in any part thereof and thus continuously controls the extraction.

A first aspect of the present invention consists of a photoconverter consisting at least of the following elements:
- at least 50 decanting, extraction and flow control towers (1) per hectare of surface used.
- at least 15 tubes for conducting photosynthesis (2) for each decanting, extraction and flow control tower (1).
- at least 20 mixture and buffer tanks (3) per hectare of surface used.
- at least 15 recirculation pumps (4) per hectare of surface used.
- at least 15 heat exchangers (5) to maintain the temperature of the photoconverter per hectare of surface used.
- at least 15 desuperheaters (6) to reduce the inlet temperature of carbon dioxide (6a), hereinafter CO₂, per hectare of surface used.
- at least 50 electromagnetic flow control valves (8) per hectare of surface used.
- at least 50 electromagnetic extraction valves (9) per hectare of surface.
- at least 50 control sensors (10) of the culture medium per hectare of surface.
- at least 50 oxygen extraction valves (11) per hectare of surface.
- at least 50 hydrogen extraction valves (12) per hectare of surface.
- at least 5 decanting tanks (14) per hectare of surface.
- 100% natural light inlets (15) of the useful surface.
- at least 100 artificial lighting lamps (16) per hectare of surface.
- at least 5 control panels (17) per hectare of surface.
- at least 5 recirculation systems (18) per hectare of surface.
- at least 2 cooling towers or condensers (19) per hectare of surface.
- at least 50 densimeters (20) per hectare of surface.
- at least 5 pumps (21) for reintroducing the liquid from (14) per hectare of surface.

The decanting, extraction and flow control towers (1) contain at least densimeters (20), oxygen extraction valves (11) located in the upper part thereof and hydrogen extraction valves (12) and they have gas sensors (13) coupled thereto, which gas sensors act such that they open at a higher gas concentration. They are transparent tubes having a diameter from 0.80 to 1.50 meters with a height from 3 to 5 meters. In addition, they are equipped with electromagnetic extraction valves (9) in the lower part thereof and with nutrient ionization injectors (7) next to them, gas extraction valves in the upper part (11) and motor-driven Tichelmann-type flow circulation valves.

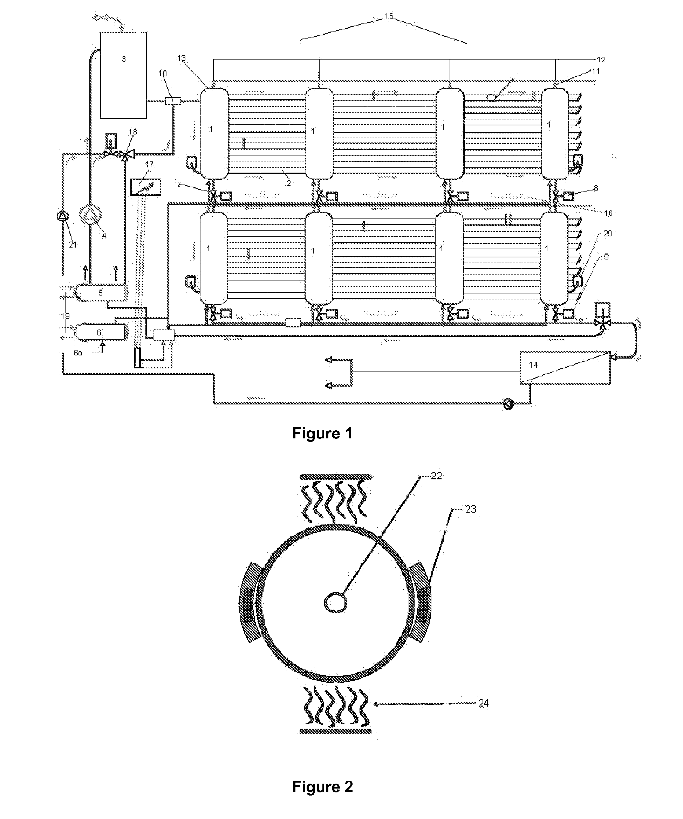

The tubes for conducting photosynthesis (2) can be horizontally or vertically arranged and are arranged perpendicular to the decanting, extraction and flow control towers (1). In addition, photosynthesis is carried out therein by means of the phytoplankton present therein and therefore these tubes are preferably made of a transparent material so as to allow the passage of electromagnetic radiation during hours of sunlight. In this same sense they contain fiber-optic (Figure 2, 22) acting as an interior artificial light diffuser. Other external elements of the tubes for conducting photosynthesis (2) are electromagnets (Figure 2, 24) which accelerate the molecular electron exchange. These tubes have a diameter comprised within the range from 100 to 200 millimeters, a length comprised within the range from 6 to 12 meters, a useful lighting surface comprised within the range from 0.30 m²/m and 0.70 m²/m and an internal volume comprised within the range from 7 l/m to 18 l/m. There is a separation of at least 250 millimeters between these tubes so as to allow light diffusion.

The mixture and buffer tanks (3) contain nutrients necessary for the development and growth of the phytoplankton mainly contained in (1) and (2). They are cylindrical shaped, made of a transparent material and have an internal volume comprised within the range from 500 to 3000 m³. With respect to the mixture and buffer tanks (3), they are made of a transparent material, are cylindrical and are arranged vertically. They have a level and nutrient inlet control using pH, temperature and concentration sensors, etc.

The culturing conditions of the phytoplankton in the photoconverters are as follows:
- Temperature: from 15 to 35°C, preferably from 20 to 30°C.
- Sunlight intensity: 600 to 800 watts/m².
- Artificial light intensity: 4 to 30 watts/m².
- Photoperiod: 12-24 hours.
- Salinity: 0 per thousand up to 50 per thousand.
- Phytoplankton concentration in the culture medium: from 1,000,000 of cells/ml up to 400,000,000 cells/ml.
- pH: from 7 to 8.4.

When referring to nutrients, these nutrients are carbon dioxide, hereinafter CO₂, NOx, vitamins, antibiotics, fungicides, water, trace elements and orthophosphoric acid.

The antibiotics added to the culture are a mixture of penicillin and streptomycin at a concentration range from 100 to 300 mg/l each, preferably at a concentration range from 150 to 250 mg/l and more preferably at a concentration of 200 mg/l for each of the components of the mixture.

The fungicides added to the culture are a mixture of griseofulvin and nystatin at a concentration range from 100 to 300 mg/l each, preferably at a concentration range from 150 to 250 mg/l and more preferably at a concentration of 200 mg/l for each of the components of the mixture.

The water added for the phytoplankton culture can be freshwater, brackish water or salt water.

The recirculation pumps (4) are centrifugal-type pumps and allow a flow rate comprised within the range from 4 to 100 cm/sec.

The heat exchangers (5) are laminar flow plate-type exchangers, are controlled by temperature sensors and are used to maintain the temperature by means of water from the cooling towers or condenser (19) of the system.

The desuperheaters (6) are laminar flow plate-type desuperheaters and have the purpose of reducing the inlet temperature of CO₂ and NOₓ (6a) coming from a pyrolysis gasifier.

Pyrolysis gasifier is understood to be a combustion process occurring at a temperature ranging between 800 and 1200°C and which allows using and recycling most of the available waste. The process allows treating segregated or mixed urban and industrial waste. It is also technically possible, without any additional difficulties, to treat toxic waste, hospital waste, tires in the same industrial plants, i.e. to propose an overall definitive solution to the problems set forth by waste. The different advantages include the fact that the process is economically viable and less expensive than any other process, and particularly, incineration, thermolysis, methanation processes, and the fact that the process is completely ecological, without any environmental impact and offers a definitive solution to the waste problem, i.e., a "zero dump" solution. The pyrolysis gasifier achieves that all the waste is transformed into synthesis gas, hydrochloric acid, hydrofluoric acid and hydrogen sulfide, the latter three being three products which can be relatively easily made inert.

A synthesis gas is understood as a gas formed by a mixture of carbon monoxide, hereinafter CO, and hydrogen, hereinafter H₂, which is obtained from a mixture of methane, hereinafter CH₄, and oxygen, hereinafter O₂, obtained from the air and steam by means of a pyrolysis gasifier.

The photoconverter can additionally include at least 50 ion sprayers (7) per hectare of surface, such that the nutrients are ionized and better and more efficient assimilation of the nutrients by the phytoplankton contained in (1) and (2) is thus allowed. Ion sprayer is understood as any system known in the state of the art that is able to ionize molecules.

The electromagnetic flow control or extraction valves (9) are located at the base of the decanting, extraction and flow control towers (1) and depend on photovalves operating by the difference of the light intensity between two points (controlled opening).

The control sensors (10) control the temperature, pH, the carbon dioxide, oxygen, trace element, antibiotic and fungicide concentration.

The photoconverter can additionally contain at least 5 gas sensors (13) per hectare of surface.

The decanting tanks (14) separate the biomass produced by the phytoplankton of the water of the culture medium. These decanting tanks are static tanks. The separated biomass contains, among other products and in no limiting sense, lipids, carbohydrates and products derived from the secondary metabolism of the phytoplankton. A transesterification reaction is carried out in the lipids present in the biomass separated by (14) such that biofuels and other energetic products are produced.

The carbohydrates obtained from the biomass separated by (14) are subjected to catalysis such that naphthas, kerosenes, polymers and gases coming from the catalysis of carbohydrates are obtained.

Transesterification refers to the chemical reaction by means of which the alkoxy group of an ester is exchanged for another alcohol, as shown in the following reaction:

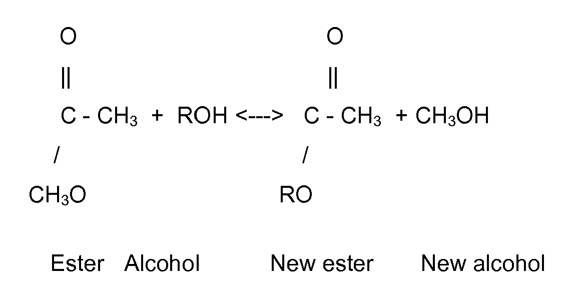

To carry out transesterification of the lipids obtained as part of the biomass produced by the phytoplankton described in this process, ethanol and methanol from a Fischer Tropsch-type reactor are used, which reactor is in turn fed by means of synthesis gas, steam and heat from a pyrolysis gasifier for biomass or waste from dumps. On the other hand, the biomass pyrolysis gasifier produces CO₂ and NOₓ which are part of the nutrients used for the feeding and growing cultured phytoplankton present in (1) and (2). The excess gases of the pyrolysis gasifier are furthermore reused to obtain electric and thermal energy, the latter being reused for desalinization.

A greenhouse-type system allows light to pass through to the natural light inlets (15), which are covered with translucent plastic, to limit the light or electromagnetic radiation intensity depending on the season of the year. In this same sense, the electromagnetic energy supplied comprises wavelengths of the spectrum ranging from 430 to 690 nm.

In addition, the decanting, extraction and flow control towers (1) and tubes for conducting photosynthesis (2) contain fiber-optic (Figure 2, 22), which is controlled by photosensors (Figure 2, 23) which in turn allow measuring the interior luminous intensity.

The control panels (17) control the injection of the different nutrients of the culture medium flow.

The recirculation systems (18) cause a Venturi-type effect which consists of the pressure of the fluid in the current of a fluid inside a closed conduit being reduced as the velocity increases when it passes through an area having a smaller section. If at this point of the conduit the end of another conduit is introduced, the fluid contained in this second conduit is suctioned for recirculation to prevent the algae from being destroyed due to the pressure.

The pumps (21) for reintroducing liquid are connected to the decanting tanks (14), such that the water separated in (14) recirculates to (3) passing through (21). Another aspect of the present invention are the products obtained by means of the photoconverter, such that mainly biofuels are obtained, in addition to pharmacopeial products such as fatty acids and lutein, among others, cosmetic products such as glycerin, pigments and emulsifying substances, industrial products with a high silica content such as borosilicates and ferrosilicates, fertilizing products, agricultural products, industrial products and livestock products, and thermal and electric energy.

### Brief Description of the Drawings

Figure 1 shows a representative diagram of the photoconverter object of the present invention with each of its parts and fittings for the use of solar and artificial electromagnetic energy for the purpose of obtaining, among other products, biofuels.
Figure 2 shows a representative diagram of a section of the tubes for conducting photosynthesis (2), with all its external and internal components.

### Embodiment

An inoculum of a phytoplankton strain is introduced in (3), culture medium and nutrients are added which are detected and regulated by means of (10) and (17). Circulation thus begins so as to establish a continuous flow within which the phytoplankton cells will travel, at the same time reproducing through (1) where they are insufflated with carbon dioxide (6a) coming from (6) which controls the temperature and are ionized in (7), and then they will move to (2), uptaking the electromagnetic energy by means of (15) and (16) and (22), in order to conduct photosynthesis.

This is where the luminous intensity is controlled by means of (23) and in (24) is where the electromagnetic field aids in polarizing the CO₂ molecules, thus aiding in their dissolution and thus increasing the biomass, rich in lipids and carbohydrates, among other products. Then it will go to the next (1), also with CO₂, with turbulence being generated and to stabilize itself, the phytoplankton tries to float in the medium. For each turbulence step, O₂ is lost by means of (11) and is detected by (13), and the same occurs for H₂ by means of (12) and is detected by (13). A recirculation process (8) is carried out within a Tichelmann-type pressure equilibrium process until the phytoplankton biomass is sufficient and detected by (20) in order to begin the extraction of part of it through (9) and from there the extracted part goes to (14), in which the water is separated from the biomass and is again propelled by (21) to the Venturi-type recirculation systems (18), finally going to (3) and to (1). The part that is not extracted due to a lack of density and therefore not detected by (20) is reintroduced into the system by (4), passing through (5) to maintain its temperature.

## Claims

1. An energy photoconverter for obtaining biofuels **characterized in that** it comprises at least the following elements:
a. decanting, extraction and flow control towers (1);
b. tubes for conducting photosynthesis (2);
c. mixture and buffer tanks (3);
d. recirculation pumps (4);
e. heat exchangers (5) to maintain the temperature of the photoconverter;
f. desuperheaters (6) to reduce the CO₂ inlet temperature (6a);
g. electromagnetic flow control valves (8);
h. electromagnetic extraction valves (9);
i. control sensors (10) of the culture medium;
j. oxygen extraction valves (11);
k. hydrogen extraction valves (12);
l. decanting tanks (14);
m. natural light inlets (15);
n. artificial lighting lamps (16);
o. control panels (17);
p. recirculation systems (18);
q. cooling towers or condensers (19);
r. densimeters (20); and
s. pumps (21) for reintroducing the liquid coming from (14)
t. electromagnets (24).

2. An energy photoconverter for obtaining biofuels according to claim 1, **characterized in that** decanting, extraction and flow control towers (1) have a diameter comprised within the range from 0.80 to 1.50 meters and a height comprised within the range from 3 to 5 meters.

3. An energy photoconverter for obtaining biofuels according to claim 1, **characterized in that** decanting, extraction and flow control towers (1) contain at least densimeters (20), oxygen extraction valves (11) and hydrogen extraction valves (12).

4. An energy photoconverter for obtaining biofuels according to claim 1, **characterized in that** the tubes for conducting photosynthesis (2) are horizontally arranged.

5. An energy photoconverter for obtaining biofuels according to claim 1, **characterized in that** the tubes for conducting photosynthesis (2) are vertically arranged.

6. An energy photoconverter for obtaining biofuels according to claim 1, **characterized in that** the tubes for conducting photosynthesis (2) contain phytoplankton.

7. An energy photoconverter for obtaining biofuels according to claim 1, **characterized in that** the mixture and buffer tanks (3) contain nutrients necessary for the development and growth of the phytoplankton.

8. An energy photoconverter for obtaining biofuels according to claim 1, **characterized in that** the mixture and buffer tanks (3) are cylindrical, made of a transparent material and have an internal volume comprised within the range from 500 to 3000 m³.

9. An energy photoconverter for obtaining biofuels according to claim 1, **characterized in that** the recirculation pumps (4) have a flow rate comprised within the range from 4 to 100 cm/sec.

10. An energy photoconverter for obtaining biofuels according to claim 1, **characterized in that** the recirculation pumps (4) are centrifugal-type pumps.

11. An energy photoconverter for obtaining biofuels according to claim 1, **characterized in that** the heat exchangers (5) are laminar flow plate-type exchangers.

12. An energy photoconverter for obtaining biofuels according to claim 1, **characterized in that** the desuperheaters (6) are laminar flow plate-type desuperheaters.

13. An energy photoconverter for obtaining biofuels according to claim 1, **characterized in that** it additionally contains ion sprayers (7).

14. An energy photoconverter for obtaining biofuels according to the previous claim, **characterized in that** the ion sprayers (7) ionize the nutrients.

15. An energy photoconverter for obtaining biofuels according to claim 1, **characterized in that** the control sensors (10) control the temperature, pH, the carbon dioxide, oxygen, trace element, antibiotic and fungicide concentration.

16. An energy photoconverter for obtaining biofuels according to claim 1, **characterized in that** it additionally contains gas sensors (13).

17. An energy photoconverter for obtaining biofuels according to claim 1, **characterized in that** the decanting tanks (14) separate the biomass from the water of the culture medium.

18. An energy photoconverter for obtaining biofuels according to claims 1 and 17, **characterized in that** the decanting tanks (14) are static-type tanks.

19. An energy photoconverter for obtaining biofuels according to claim 1 and 17 to 18, **characterized in that** the phytoplankton biomass is separated from the water through the decanting tanks (14).

20. An energy photoconverter for obtaining biofuels according to claim 1, **characterized in that** the natural light inlets (15) are covered by translucent plastic.

21. An energy photoconverter for obtaining biofuels according to claim 1, **characterized in that** the decanting, extraction and flow control towers (1) and tubes for conducting photosynthesis (2) contain fiber-optic (22).

22. An energy photoconverter for obtaining biofuels according to the previous claim, **characterized in that** the fiber-optic (22) is controlled by photosensors (23).

23. An energy photoconverter for obtaining biofuels according to the previous claim, **characterized in that** the photosensors (23) allow measuring the luminous intensity.

24. An energy photoconverter for obtaining biofuels according to claim 1, **characterized in that** the tubes for conducting photosynthesis (2) are made of a transparent material.

25. An energy photoconverter for obtaining biofuels according to claim 1, **characterized in that** the tubes for conducting photosynthesis (2) contain fiber-optic (22) which acts as an artificial light diffuser.

26. An energy photoconverter for obtaining biofuels according to claim 1, **characterized in that** the tubes for conducting photosynthesis (2) contain electromagnets (24) on the outside to accelerate the molecular electron exchange.

27. An energy photoconverter for obtaining biofuels according to claim 1, **characterized in that** the tubes for conducting photosynthesis (2) have a diameter comprised within the range from 100 to 200 millimeters, a length comprised within the range from 6 to 12 meters.

28. An energy photoconverter for obtaining biofuels according to claim 1, **characterized in that** the tubes for conducting photosynthesis (2) have a useful surface comprised within the range from 0.30 m²/m and 0.70 m²/m and an internal volume comprised within the range from 7 l/m to 18 l/m.

29. An energy photoconverter for obtaining biofuels according to claim 1, **characterized in that** the control panels (17) control the injection of the different nutrients of the culture medium flow.

30. An energy photoconverter for obtaining biofuels according to claim 1, **characterized in that** the recirculation systems (18) produce a Venturi-type effect.

31. Use of the energy photoconverter according to claim 1, for obtaining biofuels.

32. Use of the energy photoconverter according to claim 1 for obtaining pharmacopeial products such as fatty acids and lutein.

33. Use of the energy photoconverter according to claim 1 for obtaining cosmetic products such as glycerin, pigments and emulsifying substances.

34. Use of the energy photoconverter according to claim 1 for obtaining industrial products with a high silica content such as borosilicates and ferrosilicates.

35. Use of the energy photoconverter according to claim 1 for obtaining fertilizing products, agricultural products, industrial products and livestock products.

36. Use of the energy photoconverter according to claim 1 for obtaining thermal and electric energy.
